# EUROPÄISCHE PATENTSCHRIFT

(11) **EP 1 214 055 B1**
(45) Veröffentlichungstag und Bekanntmachung des Hinweises auf die Patenterteilung: **04.05.2005**
(21) Anmeldenummer: 00964183.8
(22) Anmeldetag: 16.09.2000
(51) Int. Cl.: A61K 9/06

(54) **PHARMAZEUTISCHES, WIRKSTOFFHALTIGES GEL**
PHARMACEUTICAL GEL CONTAINING ACTIVE SUBSTANCES
GEL PHARMACEUTIQUE RENFERMANT UN PRINCIPE ACTIF

(30) Priorität: 23.09.1999 DE 19945522
(43) Veröffentlichungstag der Anmeldung: 19.06.2002
(73) Patentinhaber: HEXAL AG, 83607 Holzkirchen (DE)
(72) Erfinder: BEIER, Cornelia, Hexal AG, 83607 Holzkirchen (DE); KLOKKERS, Karin, Hexal AG, 83607 Holzkirchen (DE)
(86) Internationale Anmeldenummer: PCT/EP2000/009059
(87) Internationale Veröffentlichungsnummer: WO 2001/021153

(56) Entgegenhaltungen:
- EP-A- 0 347 225
- EP-A- 0 393 904
- WO-A-00/56366
- WO-A-96/29988
- DE-A- 4 313 402

## Beschreibung

Die Erfindung betrifft ein topisch zu applizierendes, pharmazeutisches, wirkstoffhaltiges Gel mit topischer oder systemischer Wirkung, wobei auf einen sonst üblichen kurzkettigen, einwertigen Alkohol verzichtet werden kann. Neben einer sehr guten Wirksamkeit können mit einer derartigen Formulierung Hautirritationen, Hautschäden oder Hautsensibilisierungen vermieden werden.

Alkoholhaltige Gele können die Haut dort, wo sie aufgebracht werden, sensibilisieren, austrocknen oder sogar schädigen. Bei häufiger Anwendung kann eine nicht unerhebliche Beeinträchtigung der Hautschichten und damit eine ungenügende Patientencompliance die Folge sein.
Die bisher auf dem Markt befindlichen topisch zu applizierenden Gele verwenden als Lösungsmittel und Permeationsförderer mind. einen kurzkettigen, einwertigen Alkohol, meistens Ethanol, Propanol oder Isopropanol, mit einem Gehalt von mind. 20 Gew.%. Kurzkettige, einwertige Alkohole sind dafür bekannt, daß sie die Haut stark entfetten und somit austrocknen und spröde machen. Die Gele bilden zudem einen klebrigen Film auf der Haut. Dieser kann ein Spannungsgefühl beim Patienten hervorrufen und leicht unabsichtlich entfernt werden, so daß die Wirksamkeit verringert wird.

In der Literatur werden folgende topische Formulierungen beschrieben.

EP 0 393 904 offenbart eine topische Formulierung für Tretinoin mit einem Gelbildner und einem proteinartigen Material als Stabilisator für den Gelbildner. Als Gelbildner eigenen sich Carboxypolymere. Als Stabilisatoren eignen sich Proteine oder Peptide wie Kollagen und Elastin.

EP 0 347 225 beschreibt eine alkoholfreie Suspoemulsion mit Primycin, Propylenglycol und einem nicht-ionischen Tensid wie ethoxyliertem Stearylalkohol (Polawax).

WO 96/29988 offenbart eine topische Zusammensetzung mit Lecithin, Isopropylmyristat, Harnstoff, Tensid und Wasser.

DE 43 13 402 beschreibt alkoholhaltige, transdermale Wirkstoffzubereitungen.

WO 00/56366 offenbart eine Gel-Mikroemulsion mit einem Lipid (z.B. mittelkettiges Triglycerid), einem Tensid (z.B. ethoxyliertes Rhizinusöl und Phospholipid), einem Feuchthaltemittel (z.B. Propylenglycol und PEG 200) und einem Polymergel.

Die Aufgabe der vorliegenden Erfindung ist es nun, eine topisch zu applizierende Wirkstofformulierung in Form eines Gels bereitzustellen, die topisch oder systemisch wirkt, ohne Hautirritationen, Hautschäden oder eine Hautsensibilisierung zu verursachen und trotzdem eine ausreichende bzw. verbesserte Wirksamkeit zu garantieren.

Die der Erfindung zugrundeliegende Aufgabe wird nun durch ein topisch zu applizierendes, wirkstoffhaltiges Gel mit einem Gehalt an Phospholipiden in Kombination mit natürlichem Vitamin E und/oder α-Tocopherol und/ oder α-Tocopherol-Derivaten, ausgewählt aus der Gruppe bestehend aus α-Tocopherolacetat, α-Tocopherolsuccinat und α-Tocopherolpolyethylenglykolsuccinat, das keinen kurzkettigen, einwertigen Alkohol beinhaltet, gelöst.

Das erfindungsgemäße Gel bildet keinen Film auf der Haut. Es zieht gut in die Haut ein, wirkt rückfettend, pflegend und leicht kühlend. Die Haut bleibt glatt und geschmeidig. Durch eine bessere Wirkstoff-Anreicherung bzw. Permeation kann gegebenenfalls eine geringere Wirkstoffkonzentration in dem Gel eingesetzt werden als in den üblichen Darreichungsformen bei gleicher Wirksamkeit. Die bei den bisher verwendeten topisch applizierbaren Wirkstofformulierungen auftretenden unerwünschten Nebenwirkungen können mit der erfindungsgemäßen Gelformulierung verhindert werden.

Als pflegende Komponenten enthält das erfindungsgemäße Gel Phospholipide in Kombination mit natürlichem Viatmin E (Copherol), α-Tocopherol und/oder α-Tocopherol-Derivaten, ausgewählt aus der Gruppe bestehend aus α-Tocopherolacetat, α-Tocopherolsuccinat und α-Tocopherolpolyethylenglykolsuccinat. Die Phospholipide erhöhen bekanntermaßen den Feuchtigkeitsgehalt der Haut und die Permeation von Wirkstoffen durch die Haut bzw. die Anreicherung in der Haut, abhängig von den eingesetzten Wirkstoffen. Natürliches Vitamin E, die α-Tocopherol-Komponente oder die α-Tocopherol-Derivate haben zusätzlich noch eine antioxidative Wirkung, wodurch der Wirkstoff und die gesamte Formulierung stabilisiert und die Haut vor äußeren Einflüssen wie z.B. UV-Strahlung geschützt werden kann.

Die in dem erfindungsgemäßen Gel enthaltenden Wirkstoffe können Polihexanid, Lidocain, Lidocain-HCl, Oxybutinin, Tolterodin, Isosorbiddinitrat, Isosorbidmononitrat, Molsidomin und/oder Nitroglycerin sein.

Die in dem erfindungsgemäßen Gel enthaltenden Wirkstoffe können Corticosteroide, z.B. Fluocinolonacetonid, Prednisolonacetat, Prednison, Prednicarbat, Hydrocortison, Hydrocortisonbuteprat, Hydrocortison-21-acetat, Fluocinonid, Fluocortolon, Fluocortolon-21-hexanoat, Fluocortinbutyl, Betamethason, Beclomethason, Betamethason-17-valerat, Beclomethasondipropionat, Clobetasol, Clobetasol-17-propionat, Dexamethason, Budesonidbase, Budenosidpropionat, Flunisolid, Flunisolidacetat, Triarncinolon, Triamcinolonacetonid, Methylprednisolon, Fluticason, Betamethason, Deflazacort, Cortison, Cortisonacetat, Prednilyden, Cloprednol und/ oder deren Derivate, sein.

Die in dem erfindungsgemäßen Gel enthaltenden Wirkstoffe können Androgene, z.B. Testosteron, Testosteronundecanoat, Androsteron und/ oder deren Derivate, sein.

Die in dem erfindungsgemäßen Gel enthaltenden Wirkstoffe können Östrogene, z.B. Estradiol, Estradiolbenzoat, Estradiolvalerat, Estradioldipropionat, Estron, Estriol, Diethylstilbestrol, Diethylstilbestroldimethylether, Diethylstilbestroldiphosphat, Diethylstilbestroldipropionat und/ oder deren Derivate, sein.

Die in dem erfindungsgemäßen Gel enthaltenden Wirkstoffe können Gestagene, z.B. Progesteron, Norethisteron, Levonorgestrel und/ oder dessen Derivate, sein.

Die in dem erfindungsgemäßen Gel enthaltenden Wirkstoffe können Entwöhnungsmittel, z.B. Nicotin, Lobelin, Disulfiram und/ oder deren Derivate, sein.

Die in dem erfindungsgemäßen Gel enthaltenden Wirkstoffe können Analgetika, z.B. Acetylsalicylsäure, Ibuprofen, Ketoprofen, Alminoprofen, Bermoprofen, Carprofen, Dexibuprofen, Dexketoprofen, Fenoprofen, Flobufen, Flunoxaprofen, Flurbiprofen, Loxoprofen, Pelobiprofen, Pranoprofen, Pentazocin, Tilnoprofen, Ximoprofen, Zaltroprofen, Dextropropoxyphen, Phenylbutazon, Mofebutazon, Diclofenac, Aceclofenac, Amfenac, Bromfenac, Clidanac, Etodolac, Felbinac, Fentiazac, Lonazolac, Mofezolac, Oxindanac, Tifurac, Indomethacin, Acemetacin, Piroxicam, Ampiroxicam, Meloxicam, Isoxicam, Lornoxicam, Tenoxicam, Butorphanol Piritramid, Pentazocin und/ oder dessen Derivate, sein.

Die in dem erfindungsgemäßen Gel enthaltenden Wirkstoffe können Gerinnungshemmer, z.B. Heparin-Natrium, Certoparin, Dalteparin, Danaparoid, Enoxaparin, Nadroparin, Reviparin, Tinzaparin, Heparinoide und/ oder deren Derivate, sein.

Die in dem erfindungsgemäßen Gel enthaltenden Wirkstoffe können Antiparkinsonmittel, z.B. Aptiganel, Budipin, Cabergolin, Droxidopa, Etacapon, Idazoxan, Lazabemid, Milacemid, Mofegilin, Pergolid, Pramipexol, Quineloran, Rasagelin, Remacemid, Ropinorol, Selegilin, Talipexol, Tolcapon und/ oder deren Derivate, sein.

Die in dem erfindungsgemäßen Gel enthaltenden Wirkstoffe können Antihistaminika, z.B. Acrivastin, Astemizol, Desloratadin, Ebestin, Emedastin, Epinastin, Fexofenadin, Levocabastin, Loratadin, Mequitazin, Misoprostol, Mizolastin, Nafamostat, Norastemizol, Oxatomid, Tazifyllin, Temelastin, Terfenadin und/ oder deren Derivate, sein.

Die in dem erfindungsgemäßen Gel enthaltenden Wirkstoffe können Angiotensin-II-Antagonisten, z.B. Candesartan, Candesartan-Cilexetil, Eprosartan, Irbesartan, Losartan, Tasosartan, Telmisartan, Valsartan und/ oder deren Derivate, sein.

Die in dem erfindungsgemäßen Gel enthaltenden Wirkstoffe können ACE-Hemmer, z.B. Alacepril, Benazepril, Ceronapril, Cilazapril, Denapril, Enalapril, Enalaprilmaleat, Fosinopril, Imidapril, Lisinopril, Moexipril, Moveltipril, Perindopril, Quinapril, Ramipril, Ramiprilat, Rentiapril, Spirapril, Temocapril, Trandolapril, Utibapril, Zofenopril und/ oder deren Derivate, sein.

Es können in der erfindungsgemäßen Gelformulierung auch Gemische der oben genannten Wirkstoffe enthalten sein. Es können hydrophile Wirkstoffe ebenso wie lipophile Wirkstoffe eingearbeitet werden.

Die bevorzugte Gelform stellt ein Emulsionsgel dar. Die wäßrige Phase wird mit der Phospholipidphase emulgiert. Der Wirkstoff liegt gelöst in der Formulierung vor. Die Viskosität der Formulierung wird nach belieben eingestellt, indem Wasser und/ oder Glycerin und/ oder Propylenglykol mit Hilfe von Verdickungsmitteln gebunden werden.
Als Verdickungsmittel können z.B. Agar-Agar, Carrageen, Tragant, Gummi arabicum, Alginate, Pektine, Polyosen, Guar-Mehl, Johannisbrotbaumkemmehl, Stärke, Dextrine, Gelatine, Casein, Carboxymethylcellulose, Celluloseether (Hydroxyethyl-, Hydroxymethyl-, Hydroxypropyl- und Hydroxypropylmethylcellulose), Polyacryl- und Polymethacryl-Verbindungen, Vinylpolymere, Polycarbonsäuren, Polyether, Polyimine und/ oder Polyamide verwendet werden.

Der Wirkstoffgehalt kann 0,01-20 Gew. %, bezogen auf das Gesamtgewicht, betragen.

Der Gehalt an Phospholipiden beträgt 0,5-15 Gew.%, bezogen auf das Gesamtgewicht.

Der Gehalt an natürlichem Vitamin E und / oder α-Tocopherol und/ oder dessen Derivaten, ausgewählt aus der Gruppe bestehend aus α-Tocopherolacetat, α-Tocopherolsuccinat und α-Tocopherolpolyethylenglykolsuccinat, beträgt 0,01-5,0 Gew.%, bezogen auf das Gesamtgewicht.

Der Gehalt an Propylenglykol kann 1-20 Gew.%, bezogen auf das Gesamtgewicht, betragen.

Als hautpflegende bzw. rückfettende Substanzen können Fettalkohole und/ oder Fettsäurester wie z.B. 2-Octyldodecanol, Ölsäuredecylester, Miglyol 812, Cholesterolester und/ oder Panthenol in einer Konzentration von 0,5-10 Gew.%, bezogen auf das Gesamtgewicht, zugegeben werden.

Des weiteren kann das erfindungsgemäße Gel noch nach dem Stand der Technik bekannte Hilfsstoffe wie z.B. Emulgatoren, Konservierungsmittel, Stabilisatoren, Farbstoffe, Farbpigmente, Aromastoffe bzw. Duftstoffe enthalten.

Eine bevorzugte Ausführungsform der erfindungsgemäßen Gelformulierung stellt ein Emulsionsgel zur Behandlung von entzündlichen Hauterkrankungen dar, beinhaltend Phospholipide in Kombination mit natürlichem Vitamin E und/ oder α-Tocopherol und/oder α-Tocopherol-Derivaten, bevorzugt α-Tocopherolacetat, sowie mind. ein Corticosteroid. Unter entzündlichen Hauterkrankungen werden hier Erkrankungen wie atopische Dermatitis oder Neurodermitis, Psoriasis, akute und chronische Ekzeme, Verbrennungen und Kontaktekzeme verstanden.

Die topische Verabreichung von entzündungshemmenden Corticosteroiden ist in den Fällen angebracht, in denen eine hohe örtliche Konzentration der Wirkstoffe im Gewebe unter der vorgeschädigten Haut erforderlich ist, während die orale oder parenterale systemische Verabreichung zu unerwünschten Nebenwirkungen führt oder diese Verabreichungsart nicht die erforderliche örtliche Konzentration bieten kann. Wie schon oben erwähnt, können viele topische Wirkstofformulierungen die Haut dort, wo sie aufgebracht werden, sensibilisieren, austrocknen oder weiter schädigen. Gerade die durch die oben genannten entzündlichen Erkrankungen in Mitleidenschaft gezogene Haut ist sehr trocken , spröde und somit besonders empfindlich gegenüber derartigen topisch applizierbaren Wirkstofformlierungen.

Die in dem erfindungsgemäßen Gel zur Behandlung von entzündlichen Hauterkrankungen enthaltenden Corticosteroide können z.B. Fluocinolonacetonid, Prednisolonacetat, Prednison, Prednicarbat, Hydrocortison, Hydrocortisonbuteprat, Hydrocortison-21-acetat, Fluocinonid, Fluocortolon, Fluocortolon-21-hexanoat, Fluocortinbutyl, Betamethason, Beclomethason, Betamethason-17-valerat, Beclomethasondipropionat, Clobetasol, Clobetasol-17-propionat, Dexamethason, Budesonidbase, Budenosidpropionat, Flunisolid, Flunisolidacetat, Triamcinolon, Triamcinolonacetonid, Methylprednisolon, Fluticason, Betamethason, Deflazacort, Cortison, Cortisonacetat, Prednilyden, Cloprednol und/ oder deren Derivate, insbesondere Prednicarbat sein.

Der Corticosteroidgehalt kann 0,01-1,0 Gew.%, insbesondere 0,1- 0,3 Gew.%, bezogen auf das Gesamtgewicht, betragen.

Der Gehalt an Phospholipiden beträgt 1-10 Gew.%, insbesondere 1-5 Gew.%, bezogen auf das Gesamtgewicht.

Der Gehalt an natürlichem Vitamin E und / oder α-Tocopherol und/ oder dessen Derivaten, ausgewählt aus der Gruppe bestehend aus α-Tocopherolacetat, α-Tocopherolsuccinat und α-Tocopherolpolyethylenglykolsuccinat, beträgt 0,01-0,5 Gew.%, insbesondere 0,05-0,2 Gew.% und bevorzugt 0,1-0,2 Gew.%, bezogen auf das Gesamtgewicht.

Der Gehalt an Propylenglykol kann 1-20 Gew.%, insbesondere 3-10 Gew.% und bevorzugt 5 Gew.%, bezogen auf das Gesamtgewicht, betragen.

Als hautpflegende bzw. rückfettende Substanzen können z.B. 2-Octyldodecanol, Ölsäuredecylester, Miglyol 812, Cholesterolester und/ oder Panthenol in einer Konzentration von 0,5-10 Gew.%, insbesondere 1-5 Gew.% und bevorzugt 1,5 und 3 Gew.%, bezogen auf das Gesamtgewicht, zugegeben werden.

Des weiteren kann das erfindungsgemäße Gel noch nach dem Stand der Technik bekannte Hilfsstoffe wie z.B. Emulgatoren, Konservierungsmittel, Stabilisatoren, Farbstoffe, Farbpigmente, Aromastoffe bzw. Duftstoffe enthalten.

Eine weitere bevorzugte Ausführungsform der Erfindung stellt ein topisch applizierbares Gel zur systemischen Verabreichung von Hormonen, beinhaltend Phospholipide in Kombination mit natürlichem Vitamin E und/ oder α-Tocopherol und/ oder α-Tocopherol-Derivaten, bevorzugt α-Tocopherol, sowie mind. ein Östrogen, insbesondere Estradiol, dar. Der Gehalt an Estradiol kann 0,01 - 0,5 Gew.%, bezogen auf das Gesamtgewicht, betragen.

Die Erfindung wird durch nachstehende Beispiele näher erläutert ohne aber den Erfindungsumfang damit einzuschränken.

### Beispiel 1:

| | |
|---|---|
| Prednicarbat (mikronisiert) | 0,25 Gew.% |
| Phospholipid | 3,00 Gew.% |
| Propylenglykol | 5,00 Gew.% |
| dl-α-Tocopherolacetat | 0,13 Gew.% |

Die Gehaltsangaben beziehen sich auf das Gesamtgewicht der Formulierung.

Als weitere Hilfsstoffe werden Capryl-Caprinsäure-Triglycerid (Miglyol 812), Natrium-EDTA, gereinigtes Wasser, Polyacrylat (Carbopol®980NF), 2-Octyldodecanol (Eutanol G), Ölsäuredecylester (Cetiol V) und Benzylalkohol reinst in der nach dem Stand der Technik üblichen Konzentration zugegeben.

Als erster Schritt wird ein 1-prozentiges Carbopol 980 Gel hergestellt und eine entsprechende Menge an Eutanol G und Cetiol V zugegeben. Das Gel erhält eine milchig-weiße Farbe.
Nun erfolgt die Herstellung der Phospholipidlösung.
Der Wirkstoff wird in einer Mischung aus Phospholipon und Propylenglykol unter Rühren gelöst. Anschließend werden Miglyol 812 und dl-α-Tocopherolacetat hinzugefügt und ebenfalls gut verrührt. Zu der klaren Lösung wird eine Wassermenge, in welcher das Natrium-EDTA gelöst ist, zugegeben und dispergiert.

Im letzten Schritt wird die Phospholipidlösung in das Carbopol 980 Gel eingerührt und anschließend die bereits vorgemischte Lösung aus Polyethylenglykol und Benzylalkohol zugegeben.

### Beispiel 2:

| | |
|---|---|
| Prednicarbat (mikronisiert) | 0,125 Gew.% |
| Phospholipid | 3,00 Gew.% |
| Propylenglykol | 5,00 Gew.% |
| dl-α-Tocopherolacetat | 0,13 Gew.% |

Die Gehaltsangaben beziehen sich auf das Gesamtgewicht der Formulierung.

Als weitere Hilfsstoffe werden Capryl-Caprinsäure-Triglycerid (Miglyol 812), Natrium-EDTA, gereinigtes Wasser, Polyacrylat (Carbopol®980NF), 2-Octyldodecanol (Eutanol G), Ölsäuredecylester (Cetiol V) und Kaliumsorbat/Sorbinsäure in der nach dem Stand der Technik üblichen Konzentration zugegeben.

Herstellung erfolgt wie in Beispiel 1 beschrieben.

### Beispiel 3:

| | |
|---|---|
| Prednicarbat (mikronisiert) | 0,25 Gew.% |
| Phospholipon-NAT 8449 | 5,00 Gew.% |
| Propylenglykol | 3,00 Gew.% |
| dl-α-Tocopherolacetat | 0,13 Gew.% |

Die Gehaltsangaben beziehen sich auf das Gesamtgewicht der Formulierung.

Als weitere Hilfsstoffe werden Natrium-EDTA, gereinigtes Wasser, Polyacrylat (Carbopol®980NF), 2-Octyldodecanol (Eutanol G), Ölsäuredecylester (Cetiol V) und Benzylalkohol reinst in der nach dem Stand der Technik üblichen Konzentration zugegeben.

Herstellung erfolgt wie in Beispiel 1 beschrieben.

### Beispiel 4:

| | |
|---|---|
| Estradiol | 0,10 Gew.% |
| Phospholipon-NAT 8449 | 10,00 Gew.% |
| Propylenglykol | 3,00 Gew.% |
| dl-α-Tocopherolacetat | 0,125 Gew.% |

Die Gehaltsangaben beziehen sich auf das Gesamtgewicht der Formulierung.

Als weitere Hilfsstoffe werden Natrium-EDTA, gereinigtes Wasser, Polyacrylat (Carbopol®980NF), 2-Octyldodecanol (Eutanol G), Ölsäuredecylester (Cetiol V) und Benzylalkohol reinst in der nach dem Stand der Technik üblichen Konzentration zugegeben.

Herstellung erfolgt wie in Beispiel 1 beschrieben.

## Patentansprüche

1. Pharmazeutisches, topisch zu applizierendes, wirkstoffhaltiges Gel mit einer verbesserten Hautverträglichkeit, **gekennzeichnet durch** einen Gehalt an Phospholipiden in Kombination mit natürlichem Vitamin E und/oder α-Tocopherol und/ oder α-Tocopherol-Derivaten, ausgewählt aus der Gruppe bestehend aus α-Tocopherolacetat, α-Tocopherolsuccinat und α-Tocopherolpolyethylenglykolsuccinat, unter Verzicht auf einen kurzkettigen, einwertigen Alkohol.

2. Pharmazeutisches, topisch zu applizierendes, wirkstoffhaltiges Gel mit einer verbesserten Hautverträglichkeit nach Anspruch 1, **dadurch gekennzeichnet, daß** es in Form eines Emulsionsgels vorliegt.

3. Pharmazeutisches, topisch zu applizierendes, wirkstoffhaltiges Gel mit einer verbesserten Hautverträglichkeit nach einem der vorangegangenen Ansprüche, **gekennzeichnet durch** Polihexanid, Lidocain, Lidocain-HCl, Oxybutinin, Tolterodin, Isosorbiddinitrat, Isosorbitmononitrat, Molsidomin, Nitroglycerin, Corticosteroide, Androgene, Östrogene, Gestagene, Entwöhnungsmittel, Analgetika, Gerinnungshemmer, Antiparkinsonmitttel, Antihistaminika, Angiotensin-II-Antagoniosten, ACE-Hemmer und/ oder ein Gemisch aus mehreren dieser aktiven Substanzen als Wirkstoffkomponente.

4. Pharmazeutisches, topisch zu applizierendes, wirkstoffhaltiges Gel mit einer verbesserten Hautverträglichkeit nach Anspruch 3, **gekennzeichnet durch** Östrogene, insbesondere Estradiol als Wirkstoffkomponente.

5. Pharmazeutisches, topisch zu applizierendes, wirkstoffhaltiges Gel mit einer verbesserten Hautverträglichkeit nach Anspruch 3, **gekennzeichnet durch** Corticosteroide, insbesondere Prednicarbat als Wirkstoffkomponente.

6. Pharmazeutisches, topisch zu applizierendes, wirkstoffhaltiges Gel mit einer verbesserten Hautverträglichkeit nach einem der vorangegangenen Ansprüche, **gekennzeichnet durch** einen Wirkstoffgehalt von 0,01-20,0 Gew.% bezogen auf das Gesamtgewicht.

7. Pharmazeutisches, topisch zu applizierendes, wirkstoffhaltiges Gel mit einer verbesserten Hautverträglichkeit nach einem der vorangegangenen Ansprüche, **gekennzeichnet durch** einen Phospholipidgehalt von 0,5-15,0 Gew.% bezogen auf das Gesamtgewicht.

8. Pharmazeutisches, topisch zu applizierendes, wirkstoffhaltiges Gel mit einer verbesserten Hautverträglichkeit nach einem der vorangegangenen Ansprüche, **gekennzeichnet durch** einen Gehalt an natürlichem Vitamin E und/ oder α-Tocopherol und/ oder dessen Derivaten, ausgewählt aus der Gruppe bestehend aus α-Tocopherolacetat, α-Tocopherolsuccinat und α-Tocopherolpolyethylenglykolsuccinat, von 0,01-5,0 Gew.% bezogen auf das Gesamtgewicht.

9. Pharmazeutisches, topisch zu applizierendes, wirkstoffhaltiges Gel mit einer verbesserten Hautverträglichkeit nach einem der vorangegangenen Ansprüche, **gekennzeichnet durch** einen Gehalt an Propylenglykol von 1-20 Gew.% bezogen auf das Gesamtgewicht.

10. Pharmazeutisches, topisch zu applizierendes, wirkstoffhaltiges Gel mit einer verbesserten Hautverträglichkeit nach einem der vorangegangenen Ansprüche, **gekennzeichnet durch** einen Gehalt an hautpflegenden, rückfettenden Substanzen von 0,5-10 Gew.% bezogen auf das Gesamtgewicht.

11. Pharmazeutisches, topisch zu applizierendes, wirkstoffhaltiges Gel mit einer verbesserten Hautverträglichkeit nach einem der vorangegangenen Ansprüche, **gekennzeichnet durch** weitere Hilfsstoffe.

12. Pharmazeutisches, topisch zu applizierendes, wirkstoffhaltiges Gel mit einer verbesserten Hautverträglichkeit nach Anspruch 11, **gekennzeichnet durch** Verdickungsmittel, Emulgatoren, Stabilisatoren, Konservierungsmittel, Wasser, Farbstoffe, Farbpigmente, Aromen und/ oder Duftstoffe.

13. Pharmazeutisches, topisch zu applizierendes, wirkstoffhaltiges Emulsionsgel mit einer verbesserten Hautverträglichkeit nach Anspruch 1 zur Behandlung von entzündlichen Hauterkrankungen, **gekennzeichnet durch** einen Gehalt an mindestens einem Corticosteroid als Wirkstoffkomponente.

14. Pharmazeutisches, topisch zu applizierendes, wirkstoffhaltiges Emulsionsgel mit einer verbesserten Hautverträglichkeit zur Behandlung von entzündlichen Hauterkrankungen nach Anspruch 13, **gekennzeichnet durch** einen Gehalt an Phospholipiden, einen Gehalt an α-Tocopherolacetat und einen Gehalt an Prednicarbat unter Verzicht auf einen kurzkettigen, einwertigen Alkohol.

15. Pharmazeutisches, topisch zu applizierendes, wirkstoffhaltiges Emulsionsgel mit einer verbesserten Hautverträglichkeit zur Behandlung von entzündlichen Hauterkrankungen nach Anspruch 13 oder 14, **gekennzeichnet durch** einen Corticosteroidgehalt von 0,01-1,0 Gew.%, insbesondere 0,1-0,3 Gew.%, bezogen auf das Gesamtgewicht.

16. Pharmazeutisches, topisch zu applizierendes, wirkstoffhaltiges Emulsionsgel mit einer verbesserten Hautverträglichkeit zur Behandlung von entzündlichen Hauterkrankungen nach Anspruch 13, 14 oder 15, **gekennzeichnet durch** einen Phospholipidgehalt von 1-10 Gew.%, insbesondere 1-5 Gew.% bezogen auf das Gesamtgewicht.

17. Pharmazeutisches, topisch zu applizierendes, wirkstoffhaltiges Emulsionsgel mit einer verbesserten Hautverträglichkeit zur Behandlung von entzündlichen Hauterkrankungen nach Anspruch 13 bis 16, **gekennzeichnet durch** einen Gehalt an natürlichem Vitamin E und/ oder α-Tocopherol und/ oder dessen Derivaten, ausgewählt aus der Gruppe bestehend aus α-Tocopherolacetat, α-Tocopherolsuccinat und α-Tocopherolpolyethylenglykolsuccinat, von 0,01-0,5 Gew.%, insbesondere 0,05-0,2 Gew.% und bevorzugt 0,1-0,2 Gew.%, bezogen auf das Gesamtgewicht.

18. Pharmazeutisches, topisch zu applizierendes, wirkstoffhaltiges Emulsionsgel mit einer verbesserten Hautverträglichkeit zur Behandlung von entzündlichen Hauterkrankungen nach Anspruch 13 bis 17, **gekennzeichnet durch** einen Gehalt an Propylenglykol von 1-20 Gew.%, insbesondere 3 - 10 Gew. %, bezogen auf das Gesamtgewicht.

19. Pharmazeutisches, topisch zu applizierendes, wirkstoffhaltiges Emulsionsgel mit einer verbesserten Hautverträglichkeit zur Behandlung von entzündlichen Hauterkrankungen nach Anspruch 13 bis 18, **gekennzeichnet durch** einen Gehalt an hautpflegenden, rückfettenden Substanzen von 0,5-10 Gew.%, insbesondere 1 - 5 Gew. %, bezogen auf das Gesamtgewicht.

20. Pharmazeutisches, topisch zu applizierendes, wirkstoffhaltiges Emulsionsgel mit einer verbesserten Hautverträglichkeit zur Behandlung von entzündlichen Hauterkrankungen nach einem der vorangegangenen Ansprüche, **gekennzeichnet durch** Verdickungsmittel, Emulgatoren, Stabilisatoren, Konservierungsmittel, Wasser, Farbstoffe, Farbpigmente, Aromen und/ oder Duftstoffe.

## Claims

1. A pharmaceutical gel containing active ingredients with improved hypoallergenic properties for topical application identified by its content of phospholipids in combination with natural vitamin E and/or α-tocopherol and/or α-tocopherol derivatives selected from the group comprising α-tocopherol acetate, α-tocopherol succinate and α-tocopherol polyethylene glycol succinate without the use of a short chain monohydric alcohol.

2. A pharmaceutical gel containing active ingredients with improved hypoallergenic properties for topical application in accordance with Claim 1 identified by being in the form of a gel emulsion.

3. A pharmaceutical gel containing active ingredients with improved hypoallergenic properties for topical application in accordance with one of the above claims identified by its content of polihexanide, lidocaine, lidocaine-HCI, oxybutinin, tolterodine, isosorbide dinitrate, isosorbide mononitrate, molsidomine, nitroglycerin, corticosteroids, androgen, oestrogen, gestagen, weaning agents, analgesics, anticoagulants, anti-parkinson agents, antihistamines, angiotensin-II-antagonists, ACE inhibitors and/or a mixture of several of these substances as active ingredients.

4. A pharmaceutical gel containing active ingredients with improved hypoallergenic properties for topical application in accordance with Claim 3 identified by its content of oestrogen, particularly estradiol, as an active ingredient.

5. A pharmaceutical gel containing active ingredients with improved hypoallergenic properties for topical application identified by its content of corticosteroids, particularly prednicarbate, as active ingredients.

6. A pharmaceutical gel containing active ingredients with improved hypoallergenic properties for topical application in accordance with one of the above claims identified by its content of active ingredients being 0.01 - 20.0 % by weight in relation to the total weight.

7. A pharmaceutical gel containing active ingredients with improved hypoallergenic properties for topical application in accordance with one of the above claims identified by its content of phospholipids being 0.5 - 15.0 % by weight in relation to the total weight.

8. A pharmaceutical gel containing active ingredients with improved hypoallergenic properties for topical application in accordance with one of the above claims identified by its content of natural vitamin E and/or α-tocopherol and/or α-tocopherol derivatives selected from the group comprising α-tocopherol acetate, α-tocopherol succinate and α-tocopherol polyethylene glycol succinate being 0.01 - 5.0 % by weight in relation to the total weight.

9. A pharmaceutical gel containing active ingredients with improved hypoallergenic properties for topical application in accordance with one of the above claims identified by its content of propylene glycol being 1 - 20 % by weight in relation to the total weight.

10. A pharmaceutical gel containing active ingredients with improved hypoallergenic properties for topical application in accordance with one of the above claims identified by its content of skin-care, moisturising substances being 0.5 - 10 % by weight in relation to the total weight.

11. A pharmaceutical gel containing active ingredients with improved hypoallergenic properties for topical application in accordance with one of the above claims identified by its content of other supplementary agents.

12. A pharmaceutical gel containing active ingredients with improved hypoallergenic properties for topical application in accordance with Claim 11 identified by its content of thickening agents, emulsifiers, stabilisers, preservatives, water, colourants, coloured pigments, fragrances and/or scents.

13. A pharmaceutical gel emulsion containing active ingredients with improved hypoallergenic properties for topical application in accordance with Claim 1 for the treatment of inflammatory skin conditions identified by its content of at least one corticosteroid as an active ingredient.

14. A pharmaceutical gel emulsion containing active ingredients with improved hypoallergenic properties for topical application in accordance with Claim 13 for the treatment of inflammatory skin conditions identified by its content of phospholipids, α-tocopherol acetate and prednicarbate without the use of a short chain monohydric alcohol.

15. A pharmaceutical gel emulsion containing active ingredients with improved hypoallergenic properties for topical application in accordance with Claim 13 or 14 for the treatment of inflammatory skin conditions identified by its content of corticosteroids being 0.01 - 1.0 % by weight in particular 0.01 - 0.3 % by weight in relation to the total weight.

16. A pharmaceutical gel emulsion containing active ingredients with improved hypoallergenic properties for topical application in accordance with Claim 13, 14 or 15 for the treatment of inflammatory skin conditions identified by its content of phospholipids being 1 - 10 % by weight in particular 1 - 5 % by weight in relation to the total weight.

17. A pharmaceutical gel emulsion containing active ingredients with improved hypoallergenic properties for topical application in accordance with Claims 13 to 16 for the treatment of inflammatory skin conditions identified by its content of natural vitamin E and/or α-tocopherol and/or α-tocopherol derivatives selected from the group comprising α-tocopherol acetate, α-tocopherol succinate and α-tocopherol polyethylene glycol succinate being 0,01 - 0.5 % by weight in particular 0.05 - 0.2 % by weight and preferably 0.1 - 0.2 % by weight in relation to the total weight.

18. A pharmaceutical gel emulsion containing active ingredients with improved hypoallergenic properties for topical application in accordance with Claims 13 to 17 for the treatment of inflammatory skin conditions identified by its content of propylene glycol being 1 - 20 % in particular 3 - 10 % by weight in relation to the total weight.

19. A pharmaceutical gel emulsion containing active ingredients with improved hypoallergenic properties for topical application in accordance with Claims 13 to 18 for the treatment of inflammatory skin conditions identified by its content of skin-care, moisturising substances being 0.5 - 10 % by weight in particular 1 - 5 % by weight in relation to the total weight.

20. A pharmaceutical gel emulsion containing active ingredients with improved hypoallergenic properties for topical application in accordance with one of the above claims for the treatment of inflammatory skin conditions identified by its content of thickening agents, emulsifiers, stabilisers, preservatives, water, colourants, coloured pigments, fragrances and/or scents.

## Revendications

1. Gel pharmaceutique à application topique contenant des principes actifs avec une tolérance cutanée améliorée, **caractérisé par** une teneur en phospholipides en combinaison avec de la vitamine E naturelle et/ou de l'α-tocophérol et/ou des dérivés de l'α-tocophérol, sélectionnés dans le groupe constitué par l'acétate d'α-tocophérol, le succinate d'α-tocophérol et le succinate d'α-tocophérol polyéthylène glycol, en renonçant à un alcool monovalent à chaîne courte.

2. Gel pharmaceutique à application topique contenant des principes actifs avec une tolérance cutanée améliorée selon la revendication 1, **caractérisé en ce qu'**il se présente sous la forme d'un gel émulsionné.

3. Gel pharmaceutique à application topique contenant des principes actifs avec une tolérance cutanée améliorée selon l'une des revendications précédentes, **caractérisé par** les substances polyhexanide, lidocaïne, lidocaïne HCI, oxybutynine, toltérodine, dinitrate d'isosorbide, mononitrate d'isosorbide, molsidomine, nitroglycérine, des corticostéroïdes, androgènes, oestrogènes, gestagènes, agents de désintoxication, analgésiques, inhibiteurs de la coagulation, antiparkinsoniens, antihistaminiques, antagonistes de l'angiotensine II, inhibiteurs de l'ECA et/ou un mélange de plusieurs de ces substances en tant que composant du principe actif.

4. Gel pharmaceutique à application topique contenant des principes actifs avec une tolérance cutanée améliorée selon la revendication 3, **caractérisé par** des oestrogènes, en particulier estradiol en tant que composant du principe actif.

5. Gel pharmaceutique à application topique contenant des principes actifs avec une tolérance cutanée améliorée selon la revendication 3, **caractérisé par** des corticostéroïdes, en particulier prednicarbate en tant que composant du principe actif

6. Gel pharmaceutique à application topique contenant des principes actifs avec une tolérance cutanée améliorée selon l'une des revendications précédentes, **caractérisé par** une teneur en principe actif de 0,01-20,0 % poids par rapport au poids total.

7. Gel pharmaceutique à application topique contenant des principes actifs avec une tolérance cutanée améliorée selon l'une des revendications précédentes, **caractérisé par** une teneur en phospholipides de 0,5-15,0 % poids par rapport au poids total.

8. Gel pharmaceutique à application topique contenant des principes actifs avec une tolérance cutanée améliorée selon l'une des revendications précédentes, **caractérisé par** une teneur en vitamine E naturelle et/ou en α-tocophérol et/ou dérivés de l'α-tocophérol, sélectionnés dans le groupe constitué par l'acétate d'α-tocophérol, le succinate d'α-tocophérol et le succinate d'α-tocophérol polyéthylène glycol de 0,01-5,0 % poids par rapport au poids total.

9. Gel pharmaceutique à application topique contenant des principes actifs avec une tolérance cutanée améliorée selon l'une des revendications précédentes, **caractérisé par** une teneur en propylène glycol de 1-20% poids par rapport au poids total.

10. Gel pharmaceutique à application topique contenant des principes actifs avec une tolérance cutanée améliorée selon l'une des revendications précédentes, **caractérisé par** une teneur en substances traitantes et reconstituantes de 0,5-10 % poids par rapport au poids total.

11. Gel pharmaceutique à application topique contenant des principes actifs avec une tolérance cutanée améliorée selon l'une des revendications précédentes, **caractérisé par** d'autres substances auxiliaires.

12. Gel pharmaceutique à application topique contenant des principes actifs avec une tolérance cutanée améliorée selon la revendication 11, **caractérisé par** des épaississants, émulsifiants, stabilisants, agents de conservation, eau, colorants, pigments colorés, arômes et/ou parfums.

13. Gel émulsionné pharmaceutique à application topique contenant des principes actifs avec une tolérance cutanée améliorée selon la revendication 1 pour le traitement des maladies cutanées inflammatoires, **caractérisé par** une teneur d'au moins un corticostéroïde en tant que composant du principe actif.

14. Gel émulsionné pharmaceutique à application topique contenant des principes actifs avec une tolérance cutanée améliorée pour le traitement des maladies cutanées inflammatoires selon la revendication 13, **caractérisé par** une teneur en phospholipides, une teneur en acétate d'α-tocophérol et une teneur en prednicarbate, en renonçant à un alcool monovalent à chaîne courte.

15. Gel émulsionné pharmaceutique à application topique contenant des principes actifs avec une tolérance cutanée améliorée pour le traitement des maladies cutanées inflammatoires selon la revendication 13 ou 14, **caractérisé par** une teneur en corticostéroïdes de 0,01-1,0 % poids, en particulier 0,1-0,3 % poids par rapport au poids total.

16. Gel émulsionné pharmaceutique à application topique contenant des principes actifs avec une tolérance cutanée améliorée pour le traitement des maladies cutanées inflammatoires selon la revendication 13, 14 ou 15, **caractérisé par** une teneur en phospholipides de 1-10 % poids, en particulier 1-5% poids par rapport au poids total.

17. Gel émulsionné pharmaceutique à application topique contenant des principes actifs avec une tolérance cutanée améliorée pour le traitement des maladies cutanées inflammatoires selon les revendications 13 à 16, **caractérisé par** une teneur en vitamine E naturelle et/ou en α-tocophérol et/ou dérivés de l'α-tocophérol, sélectionnés dans le groupe constitué par l'acétate d'α-tocophérol, le succinate d'α-tocophérol et le succinate d'α-tocophérol polyéthylène glycol, de 0,01-0,5% poids, en particulier 0,05-0,2 % poids et de préférence 0,1-0,2 % poids par rapport au poids total.

18. Gel émulsionné pharmaceutique à application topique contenant des principes actifs avec une tolérance cutanée améliorée pour le traitement des maladies cutanées inflammatoires selon les revendications 13 à 17, **caractérisé par** une teneur en propylène g lycol de 1-20% poids, en particulier 3-10 % poids par rapport au poids total.

19. Gel émulsionné pharmaceutique à application topique contenant des principes actifs avec une tolérance cutanée améliorée pour le traitement des maladies cutanées inflammatoires selon les revendications 13 à 18, **caractérisé par** une teneur en substances traitantes et reconstituantes de 5-10 % poids, en particulier 1-5 % poids par rapport au poids total.

20. Gel émulsionné pharmaceutique à application topique contenant des principes actifs avec une tolérance cutanée améliorée pour le traitement des maladies cutanées inflammatoires selon l'une des revendications précédentes, **caractérisé par** des épaississants, émulsifiants, stabilisants, agents de conservation, eau, colorants, pigments colorés, arômes et/ou parfums.
